# NOUVEAU FASCICULE DE BREVET EUROPEEN

(11) **EP 0 403 351 B2**
(45) Date de publication et mention de la décision concernant l'opposition: **23.08.2000**
(45) Mention de la délivrance du brevet: 09.08.1995
(21) Numéro de dépôt: 90401592.2
(22) Date de dépôt: 11.06.1990
(51) Int. Cl.: C07D 307/62

(54) **Procédé de préparation de l'acide ascorbique**
Verfahren zur Herstellung von Ascorbinsäure
Process for the preparation of ascorbic acid

(30) Priorité: 12.06.1989 FR 8907716
(43) Date de publication de la demande: 19.12.1990
(73) Titulaire: RHONE-POULENC BIOCHIMIE, 92160 Antony (FR)
(72) Inventeur: Le Fur, Isidore, F-94320 Thiais (FR); Richard, Jean-Paul, F-91100 Créteil (FR); Wolff, Gérard, F-94320 Thiais (FR)
(74) Mandataire: Becker, Philippe

(56) Documents cités:
- EP-A- 0 091 134
- FR-A- 779 883
- FR-A- 922 949
- JP-A- 53 098 925
- PL-A- 130 761
- US-A- 2 265 121
- CHEMICAL ABSTRACTS, vol. 90, no. 5, 29 janvier 1979, résumé no. 39204c,Columbus, Ohio, US; & JP-A-53 098 925 (TAKEDA CHEM. IND., LTD) 29-08-1978
- JOURNAL OF PHARMACEUTICAL SCIENCES, vol. 61, no. 8, août 1972, pages 1333-1334,Washington, DC, US; S.L. BAFNA et al.: "Separation of ascorbic acid and 2-keto-L-gulonic acid"
- J. Chem. Eng. Data (1984), 29, pp. 452-454
- Chemia Stosowana (1987), XXXI, 2, pp. 249-265

## Description

La présente invention concerne la préparation de l'acide ascorbique à partir de l'acide céto-2 L gulonique éventuellement sous forme de sel.

Il est connu, par exemple d'après le brevet français FR 922 949, de préparer l'acide ascorbique à partir des esters de l'acide céto-2 L gulonique (2KLG-H) en passant intermédiairement par un sel alcalin de l'acide ascorbique. Cependant la mise en oeuvre de ce procédé ne permet pas d'obtenir l'acide ascorbique avec une pureté suffisante.

Il a maintenant été trouvé, et c'est ce qui fait l'objet de la présente invention, que l'acide ascorbique pratiquement pur peut être obtenu après cristallisation à partir d'une solution hydro-méthanolique d'acide ascorbique obtenue après
a) estérification de l'acide céto-2 L ,gulonique, éventuellement sous forme de sel de sodium, en présence d'un alcool et d'un acide fort choisi parmi les acides sulfurique, chlorhydrique ou sulfonique,
b) transformation, éventuellement in situ, de l'ester de l'acide céto-2 L gulonique en ascorbate de sodium au moyen d'une base minérale ou organique en solution alcoolique,
c) séparation de l'ascorbate de sodium qui précipite,
d) déplacement de l'acide ascorbique de son sel au moyen d'acide sulfurique, en milieu hydro-méthanolique contenant jusqu'à 40% en poids d'eau,
e) séparation du sel alcalin de l'acide fort,
f) purification de l'acide ascorbique, à partir de la solution hydro-méthanolique obtenue après séparation du sel alcalin de l'acide fort, sur résine acide puis résine basique et traitement au noir décolorant, et
g) cristallisation de l'acide ascorbique pur directement à partir de la solution hydro-méthanolique obtenue après traitement au noir décolorant, puis séparation.

S.L. Bafna et coll., J. Pharmaceut. Sciences, 61, 1333 (1979) enseignent la possibilité de séparer l'acide ascorbique de l'acide céto-2 L gulonique au moyen de résines échangeuses de cations. Chem. Abst. (1979), vol. 90, n^{º} 39204c enseigne le traitement des eaux-mères de cristallisation de l'acide céto-2 L gulonique par une résine échangeuse de cations afin d'obtenir l'acide ascorbique avec une pureté de 98,2 %. Ces documents ne font pas état de la pureté ionique finale de l'acide ascorbique.

Selon la présente invention, l'acide céto-2 L gulonique, éventuellement sous forme de sel, est estérifié au moyen d'un alcool en présence d'un acide fort, puis l'ester obtenu est transformé, éventuellement in situ, par action d'une base en solution alcoolique en quantité nécessaire à la consommation quantitative de l'ester de l'acide céto-2 L gulonique, en sel de métal alcalin de l'acide ascorbique qui est isolé puis neutralisé par action d'un acide fort en opérant dans un solvant dans lequel le sel alcalin de l'acide fort est insoluble et l'acide ascorbique est soluble. Après séparation du sel alcalin de l'acide fort, l'acide ascorbique est isolé de sa solution.

L'estérification de l'acide céto-2 L gulonique est réalisée au moyen d'un alcool tel que le méthanol ou le butanol en présence d'un acide fort tel que l'acide chlorhydrique, l'acide sulfurique ou les acides sulfoniques. L'estérification est effectuée en présence d'une quantité d'acide fort qui est, de préférence, voisine de 2 % molaire par rapport au 2KLG-H mis en oeuvre ou en contrôlant le pH apparent du milieu qui est maintenu au voisinage de 0,5 lorsqu'on utilise l'acide chlorhydrique ou l'acide sulfurique.

En utilisant un sel alcalin de l'acide céto-2 L gulonique, de préférence le sel de sodium, il est nécessaire d'utiliser une quantité d'acide fort permettant la libération de l'acide céto-2 L gulonique in situ et la catalyse de la réaction d'estérification.

Il peut être avantageux d'effectuer l'estérification au moyen de butanol. En effet le butanol forme avec l'eau un azéotrope qui peut être distillé au cours de l'estérification. Il est donc ainsi possible de déplacer l'équilibre dans le sens de l'estérification.

L'utilisation du butanol permet d'effectuer l'estérification sur une solution aqueuse brute d'acide céto-2 L gulonique obtenue à partir d'un moût de fermentation productrice d'acide céto-2 L gulonique débarrassé de la biomasse et concentré.

Lorsqu'un sel alcalin de l'acide céto-2 L gulonique est mis en oeuvre, il peut être avantageux de séparer le sel alcalin de l'acide fort avant de réaliser la transformation in situ de l'ester de l'acide céto-2 L gulonique en sel alcalin de l'acide ascorbique. Toutefois, la séparation du sel alcalin de l'acide fort est nécessaire lorsque l'on désire isoler l'ester de l'acide céto-2 L gulonique après concentration de sa solution alcoolique.

Généralement l'estérification est effectuée à une température comprise entre 50 et 70°C, de préférence voisine de 65°C.

La lactonisation de l'ester de l'acide céto-2 L gulonique est effectuée généralement au moyen d'une base choisie parmi les bases minérales (soude) ou organique (méthylate de sodium) en solution alcoolique qui est ajoutée à un débit tel que la base soit immédiatement consommée et soit apportée en quantité nécessaire à la consommation quantitative de l'ester de l'acide céto-2 L gulonique. Il est particulièrement avantageux de régler le débit de la solution alcoolique de la base en fonction de la valeur du pH et d'arrêter l'addition lorsque le pH devient voisin de 8,5.

Généralement la lactonisation est effectuée à une température comprise entre 50 et 70°C, de préférence voisine de 65°C.

Après abaissement de la température au voisinage de 30°C, le sel de métal alcalin de l'acide ascorbique (ascorbate de sodium), qui peut contenir jusqu'à 10 % en poids du sel alcalin de l'acide céto-2 L gulonique (2KLG-Na) est séparé par filtration.

Le sel alcalin de l'acide ascorbique (ascorbate de sodium) contenant éventuellement du sel alcalin de l'acide céto-2 L gulonique (2KLG-Na) est neutralisé par action d'un acide fort (acide sulfurique) en opérant en solution hydro-méthanolique pouvant contenir jusqu'à 40 % en poids d'eau de préférence 15 à 25 % dans laquelle le sel alcalin de l'acide fort (sulfate de sodium) est peu soluble.

Il est particulièrement avantageux d'ajouter l'acide fort pur ou dilué à un débit constant réglé en fonction du pH de façon à neutraliser le sel alcalin de l'acide ascorbique et au moins partiellement le sel alcalin de l'acide céto-2 L gulonique. Généralement, le pH en fin d'acidification est compris entre 1,5 et 3,5 et, de préférence voisin de 3.

Le sel alcalin de l'acide fort et éventuellement le sel alcalin de l'acide céto-2 L gulonique non neutralisé sont séparés par filtration. L'acide céto-2 L gulonique peut être récupéré par lavage du gâteau de filtration par un acide fort (acide sulfurique) en solution méthanolique et être ainsi recyclé dans la phase d'estérification.

De la solution hydro-méthanolique, obtenue après séparation du sel alcalin de l'acide fort et du sel alcalin de l'acide céto-2 L gulonique non hydrolysé, l'acide ascorbique pur est isolé selon la méthode suivante :
la solution est filtrée sur une colonne de résine sulfonique acide puis sur une colonne de résine basique (type amine tertiaire) afin d'éliminer le sel alcalin d'acide fort résiduel puis décolorée par traitement au moyen de noir décolorant. La solution purifiée est ensuite concentrée sous pression réduite afin de cristalliser l'acide ascorbique pur qui est séparé par filtration.
   Les filtrats, après traitement approprié, peuvent être recyclés à l'étape d'estérification ou d'acidification.

Les filtrats de cristallisation de l'acide ascorbique pur peuvent, après traitement approprié et selon leur composition, être recyclés à l'étape d'estérification, de lactonisation ou d'acidification.

Les exemples suivants montrent comment l'invention peut être mise en pratique.

### EXEMPLE 1

Dans un réacteur de 3 litres, on introduit 1925 g soit 2436 cm3 de méthanol (60,1 moles) et 450 g de 2KLG-H, 2H₂O pur (2,12 moles). Le mélange est agité à 65°C jusqu'à dissolution complète. On ajoute alors 4,4 g soit 2,39 cm3 d'acide sulfurique à 97 % (0,043 mole) puis poursuit l'agitation pendant 4 heures 30 minutes à 65°C.

Le rendement, dosé, en ester méthylique de l'acide céto-2 L gulonique est de 96%.

La solution obtenue (2379 g) qui contient 17,7 % en poids d'ester méthylique de l'acide céto-2 L gulonique soit 423,3 g (2,03 moles) et 0,007 % d'acide céto-2 L gulonique soit 16,5 g (0,085 mole) est introduite dans un réacteur de 3 litres et est maintenue à 65°C. On ajoute, à 65°C, en 2 heures 30 minutes, 548 g soit 590 cm3 d'une solution méthanolique de soude 3,7N (2,2 moles de soude) en respectant les débits suivants :
98,8 % du volume de soude est introduit au débit de 230 cm3/heure lorsque le pH est inférieur à 8,5,
1,2 % du volume de soude est introduit au débit de 80 cm3/heure lorsque le pH est supérieur à 8,5.

L'addition de soude est arrêtée lorsque le pH devient supérieur à 8,7.

Après refroidissement à 30°C, la bouillie obtenue est filtrée. Le gâteau de filtration est lavé par 3 fois 225 cm3 de méthanol à une température voisine de 20°C.

Après séchage, on obtient 404,1 g d'un produit contenant :
- 92 % en poids d'ascorbate de sodium soit 371,7 g (1,88 mole)
- 8,7 % en poids de sel de sodium de l'acide céto-2 L gulonique soit 35,2 g (0,16 mole).

### EXEMPLE 2

Dans un réacteur de 3 litres, on introduit 1270 g soit 1607 cm3 de méthanol (39,7 moles), 317,5 g d'eau (17,6 moles) et 388,2 g d'ascorbate de sodium, titrant 91,9 % en poids d'ascorbate de sodium (soit 356,7 g) et 8,5 % en poids de sel de sodium de l'acide céto-2 L gulonique (soit 33 g) obtenu dans les conditions de l'exemple 1.

Le mélange réactionnel est agité à une température voisine de 20°C puis est acidifié par 98,7 g d'acide sulfurique à 97 % (56,6 cm3 ; 0,977 mole) qui est additionné en 1 heure à raison de 98 % du volume d'acide sulfurique à un débit de 60 cm3/heure (pH supérieur à 3,6) et de 2 % du volume d'acide sulfurique à un débit de 6 cm3/heure (pH inférieur à 3,6). L'addition d'acide sulfurique est arrêtée lorsque le pH est inférieur à 3.

Au cours de l'addition de l'acide sulfurique, la température de la masse réactionnelle doit être maintenue à 40°C.

Le sulfate de sodium qui a précipité est séparé par filtration à 40°C et est lavé par 474g de méthanol.

Le filtrat et les lavages sont réunis. On obtient ainsi une solution hydro-méthanolique (2273 g) contenant :
- 13,85 % en poids d'acide ascorbique soit 314,9 g (1,79 mole)
- 0,95 % en poids d'acide céto-2 L gulonique soit 21,62 g (0,11 mole)
- 0,16 % en poids de sulfate de sodium soit 3,6 g (0,025 mole).

### EXEMPLE 3

Dans les conditions de l'exemple 2, on prépare une solution hydrométhanolique d'acide ascorbique (2042 g) contenant 14,97 % en poids d'acide ascorbique soit 305,7 g (1,74 mole) et 0,8 % en poids d'acide céto-2 L gulonique soit 16,33 g (0,084 mole).

La solution est concentrée sous pression réduite à 48°C dans un cristallisoir alimenté en continu qui contient initialement un pied saturé en acide ascorbique en présence de 4 g d'amorce.

Lorsque la concentration est terminée, la bouillie est filtrée à 30°C. Le gâteau de filtration est lavé par 220 cm3 de méthanol (174 g) puis séché sous pression réduite à une température voisine de 20°C. On obtient ainsi 249,1 g d'un produit contenant 99 % en poids d'acide ascorbique.

### EXEMPLE 4

Dans un réacteur de 3 litres, on introduit 665 g soit 842 cm3 de méthanol (20,8 moles) et 153,7 g d'acide céto-2 L gulonique brut titrant 78,6 %.

Le mélange réactionnel est chauffé à 65°C sous agitation jusqu'à dissolution complète.

On ajoute alors 1,2 g d'acide sulfurique à 97 % (0,65 cm3, 0,012 mole) puis on agite pendant 6 heures à 65°C.

La solution obtenue (842,6 g) qui contient 14 % en poids d'ester méthylique de l'acide céto-2 L gulonique soit 118 g (0,56 mole), 1,5 % en poids d'acide céto-2 L gulonique soit 77 g (0,067 mole) et 0,7 % en poids d'acide ascorbique soit 6 g (0,034 mole) est concentrée à 40°C sous pression réduite (300 mm de mercure ; 39,9 kPa) pendant 2 heures. Après refroidissement, le produit qui précipite est séparé par filtration. On obtient ainsi 78,4 g d'un produit contenant 98,3 % en poids d'ester méthylique de l'acide céto-2 L gulonique soit 77 g (0,37 mole) et 1,7 % d'acide céto-2 L gulonique soit 6 g (0,007 mole).

Le filtrat (398 g), qui contient 16 % en poids d'ester méthylique de l'acide céto-2 L gulonique soit 63 g (0,30 mole), 4,4 % en poids d'acide céto-2 L gulonique soit 17,5 g (0,09 mole) et 1,6 % en poids d'acide ascorbique soit 6,5 g (0,037 mole), est concentré à 40°C sous pression réduite (300 mm de mercure; 39,9 kPa) pendant 2 heures. Après refroidissement le produit qui précipite est séparé par filtration. Après séchage, on obtient 27 g d'un produit contenant 96,3 % en poids d'ester méthylique de l'acide céto-2 L gulonique soit 26 g (0,125 mole) et 3,5 % en poids d'acide céto-2 L gulonique soit 0,95 g (0,005 mole).

### EXEMPLE 5

Dans un réacteur de 2 litres, on introduit 197,5 g soit 250 cm3 de méthanol (6,17 moles) et 52 g d'ester méthylique de l'acide céto-2 L gulonique (0,25 mole). Le mélange réactionnel agité est chauffé à 65°C. On ajoute alors 21 g de bicarbonate de sodium (0,25 mole). Le dégagement de gaz carbonique cesse après 2 heures 40 minutes. On maintient l'agitation à 65°C pendant une durée totale de 4 heures 15 minutes. Après refroidissement, à une température voisine de 20°C, le réacteur est vidangé et rincé par 347,4 g de méthanol. La bouillie est concentrée à sec sous pression réduite. On obtient ainsi 51,5 g d'un produit contenant 65,3 % en poids d'ascorbate de sodium soit 33,63 g (0,19 mole).

### EXEMPLE 6

Dans un réacteur de 500 cm3, on introduit 218,7 g soit 270 cm3 de n.butanol (2,95 moles) et 50 g d'acide céto-2 L gulonique brut titrant 83,5 %. On ajoute 2,25 g d'acide p.toluènesulfonique (0,013 mole).

Le mélange réactionnel est agité. Le butanol est évaporé sous pression réduite (110 mm de mercure; 14,6 kPa) à 70°C. Le niveau est maintenu constant dans le réacteur par addition de butanol pur. Le milieu devient homogène après 3 heures 30 minutes de chauffage. Le rendement de l'estérification est de 90 %.

L'ester butylique de l'acide céto-2 L gulonique est cristallisé de la solution butanolique par refroidissement. Après filtration, l'ester butylique de l'acide céto-2 L gulonique est lavé puis séché.

### EXEMPLE 7

Dans une ampoule de coulée à double enveloppe, on introduit 183 g soit 226 cm3 de n.butanol (2,47 moles) et 39,8 g d'ester butylique de l'acide céto-2 L gulonique à 97 % (0,154 mole) et maintient la solution à 70°C.

Dans une seconde ampoule de coulée à double enveloppe, on introduit 48 g soit 59 cm3 de n.butanol et 6,16 g de soude pure (0,154 mole).

Dans le réacteur, on introduit 13,8 g de n.butanol soit 17 cm3 (0,18 mole), 1,66 g d'acide ascorbique pur (0,009 mole) et 0,51 g de méthylate de sodium (0,009 mole).

On ajoute simultanément, en 2 heures, le contenu des deux ampoules de coulée en maintenant la température du réacteur à 62°C.

L'ascorbate de sodium précipite au cours de la réaction.

Après refroidissement à une température voisine de 20°C, l'ascorbate de sodium est séparé par filtration et est lavé par 97 g soit 120 cm3 de n.butanol et 198 g soit 300 cm3 de n.hexane.

On obtient ainsi 31,8 g d'un produit contenant 85,5 % d'ascorbate de sodium soit 0,137 mode.

### EXEMPLE 8

Dans un réacteur de 2 litres, on introduit 395 g soit 500 cm3 de méthanol, 100 g d'acide céto-2 L gulonique monohydraté pur (0,471 mole) et 2 cm3 d'acide chlorhydrique 12N soit 2,4g. On chauffe sous agitation pendant 2 heures à 65°C. On neutralise par addition de 2,5 cm3 de soude 10N.

Après concentration à sec sous pression réduite, on obtient 100,3 g d'un produit contenant 86,7 % en poids d'ester méthylique de l'acide céto-2 L gulonique (0,417 mole).

### EXEMPLE 9

Dans un réacteur de 500 cm3, on introduit 158 g soit 200 cm3 de méthanol (4,9 moles), 12,41 g soit 6,74 cm3 d'acide sulfurique à 97 % (0,123 mole) et 44,35 g de sel de sodium de l'acide céto-2 L gulonique titrant 83,3 % (0,171 mole).

Le mélange agité est chauffé à 65°C pendant 4 heures 30 minutes. On ajoute 11,5 cm3 de soude méthanolique 3,5N de façon à amener le pH à 4.

Le sulfate de sodium qui a précipité est séparé par filtration et est lavé par 100 g soit 126 cm3 de méthanol.

Le filtrat et les lavages sont réunis. On obtient ainsi une solution contenant 32,7 g d'ester méthylique de l'acide céto-2 L gulonique.

### EXEMPLE 10

Dans un réacteur de 3 litres, on introduit 2032,8 g d'une solution contenant 1358,5 g de méthanol (42,4 moles), 336,5 g d'eau (18,7 g), 312,6 g d'acide ascorbique (1,77 mole), 6,5 g d'acide céto-2 L gulonique (0,033 mole) et 18,7 g d'ester méthylique de l'acide céto-2 L gulonique (0,09 mole).

La solution est envoyée sur des colonnes de résines montées en série : la première contenant 220 cm3 de résine sulfonique (Amberlite^{R} IRN 77), la seconde contenant 110 cm3 de résine basique (Amberlite^{R} A368) à 40°C au débit de 3 litres/heure.

Les résines sont lavées par 316 g de méthanol.

Le filtrat et les lavages sont réunis. On ajoute une bouillie de noir décolorant constituée de 118 g de méthanol (149 cm3) et de 5 g de noir décolorant (Acticarbon^{R} L3S préalablement traité à l'acide sulfurique).

La solution est agitée pendant 15 minutes à 40°C. Le noir est séparé par filtration et est lavé par 316 g de méthanol (400 cm3).

Le filtrat et les lavages sont réunis. On obtient ainsi 2717 g d'une solution contenant 1994 g de méthanol, 423 g d'eau, 274,7 g d'acide ascorbique (1,56 moles), 8,4 g d'acide céto-2 L gulonique (0,043 mole) et 16,8 g d'ester méthylique de l'acide céto-2 L gulonique.

Dans un réacteur de 3 litres, on introduit une solution (1752,9 g), obtenue dans les conditions décrites précédemment, contenant 1159 g de méthanol, 290 g d'eau, 303,8 g d'acide ascorbique (1,72 mode) et 20,86 g d'acide céto-2 L gulonique (0,107 mole).

Dans un cristallisoir, on prépare une suspension constituée de 19 g de méthanol, de 43 g d'eau et de 35 g (0,2 mode) d'acide ascorbique pur.

On agite sous pression réduite (85 mm de mercure; 11,4 kPa) à 40°C puis on ajoute la solution hydrométhanolique de façon à maintenir la température à 40°C.

Lorsque la cristallisation est terminée, la bouillie est filtrée à 40°C. le gâteau de filtration est lavé par 210 g de méthanol (266 cm3) à 40°C.

Après séchage sous pression réduite à une température voisine de 20°C, on obtient 170,0 g d'acide ascorbique pur.

## Revendications

1. Procédé de préparation d'acide ascorbique pur à partir d'acide céto-2 L-gulonique, comprenant les étapes suivantes :
a) estérification de l'acide céto-2 L-gulonique, éventuellement sous forme de sel de sodium, en présence **d'un alcool et** d'un acide fort choisi parmi les acides sulfurique, chlorhydrique ou sulfonique,
b) transformation, éventuellement in situ, de l'ester de l'acide céto-2 L-gulonique en ascorbate de sodium, au moyen d'une base minérale ou organique en solution alcoolique,
c) séparation de l'ascorbate de sodium qui précipite,
d) déplacement de l'acide ascorbique de son sel au moyen d'acide sulfurique, en milieu hydro-méthanolique contenant jusqu'à 40% en poids d'eau,
e) séparation du sel alcalin de l'acide fort,
f) purification de l'acide ascorbique, à partir de la solution hydro-méthanolique obtenue après séparation du sel alcalin de l'acide fort, sur résine acide puis résine basique et traitement au noir décolorant, et
g) cristallisation de l'acide ascorbique pur directement à partir de la solution hydro-méthanolique obtenue après traitement au noir décolorant, puis séparation.

## Claims

1. Process for preparing pure ascorbic acid from 2-keto-L-gulonic acid, comprising the following steps:
a) esterification of 2-keto-L-gulonic acid, optionally in the form of a sodium salt, in the presence of an alcohol and of a strong acid selected from sulphuric, hydrochloric and sulphonic acids,
b) conversion of the 2-keto-L-gulonic acid ester, optionally in situ, to sodium ascorbate by means of an inorganic or organic base in alcoholic solution,
c) separation of the sodium ascorbate which precipitates,
d) displacement of the ascorbic acid from its salt by means of sulphuric acid, in an aqueous-methanolic medium containing up to 40% of water,
e) separation of the alkaline salt of the strong acid,
f) purification of the ascorbic acid, from the aqueous-methanolic solution obtained after separation of the alkaline salt of the strong acid, on an acidic resin and then on a basic resin and treatment with decolorizing charcoal, and
g) crystallization of the pure ascorbic acid directly from the aqueous-methanolic solution obtained after the treatment with decolorizing charcoal, followed by separation.

## Patentansprüche

1. Verfahren zur Herstellung von reiner Ascorbinsäure ausgehend von 2-Keto-L-Gulonsäure, umfassend die folgenden Stufen:
a) Veresterung von 2-Keto-L-Gulonsäure, gegebenenfalls in Form des Natriumsalzes, in Anwesenheit eines Alkohols und einer starken Säure, ausgewählt unter Schwefelsäure, Chlorwasserstoffsäure oder Sulfonsäure,
b) Umwandlung, gegebenenfalls in situ, des Esters der 2-Keto-L-Gulonsäure in Natriumascorbat mit Hilfe einer Mineralbase oder organischen Base in alkoholischer Lösung,
c) Abtrennung des ausfallenden Natriumascorbats,
d) Freisetzung der Ascorbinsäure aus ihrem Salz mit Hilfe von Schwefelsäure im wäßrig-methanolischen Medium, das bis zu 40 % Wasser enthält,
e) Abtrennung des Alkalisalzes der starken Säure,
f) Reinigung der Ascorbinsäure, ausgehend von der nach Abtrennung des Alkalisalzes der starken Säure erhaltenen wäßrig-methanolischen Lösung über saurem Harz und anschließend über basischem Harz sowie Behandlung mit Entfärbungskohle, und
g) Kristallisation der reinen Ascorbinsäure direkt ausgehend von der nach der Behandlung mit Entfärbungskohle und anschließender Abtrennung erhaltenen wäßrig-methanolischen Lösung.
